(19) **Europäisches Patentamt**
**European Patent Office**
**Office européen des brevets**

(11) **EP 2 997 889 B1**

(12) **EUROPEAN PATENT SPECIFICATION**

(45) Date of publication and mention
of the grant of the patent:
**29.12.2021 Bulletin 2021/52**

(51) Int Cl.:
*A61B 5/021* (2006.01)   *A61B 5/022* (2006.01)

(21) Application number: **14184913.3**

(22) Date of filing: **16.09.2014**

(54) **Blood pressure cuff with tapered bladder**

Blutdruckmanschette mit konischer Blase

Brassard de tensiomètre à vessie conique

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**23.03.2016 Bulletin 2016/12**

(73) Proprietors:
• **Gloth, Sean**
**Clarksville, MD 21029 (US)**
• **Gloth, Christopher**
**Clarksville, MD 21029 (US)**

(72) Inventors:
• **Gloth, Sean**
**Clarksville, MD 21029 (US)**
• **Gloth, Christopher**
**Clarksville, MD 21029 (US)**

(74) Representative: **Haseltine Lake Kempner LLP
Redcliff Quay
120 Redcliff Street
Bristol BS1 6HU (GB)**

(56) References cited:
**EP-A1- 0 274 735      JP-A- 2009 072 278
US-A- 4 901 732        US-A1- 2004 092 833
US-A1- 2008 027 336    US-A1- 2013 109 981**

**Description**

**FIELD OF THE INVENTION**

This invention relates generally to blood pressure measurement, and more particularly to a blood pressure cuff (sphygmomanometer) that provides accurate measurements over a range of patient arm circumferences.

**BACKGROUND**

A patient's blood pressure is measured in almost all cases using a sphygmomanometer, or blood pressure cuff. This term refers to a device consisting of an inflatable bladder housed within a sleeve (cuff) and connected to a manometer. The cuff is wrapped around the patient's upper arm and the bladder inflated to restrict blood flow through the patient's brachial artery. The manometer is used to read the pressure at which the blood flow first resumes (known as the systolic blood pressure, or SBP) and the pressure at which the blood flow becomes unimpeded (known as the diastolic blood pressure, or DBP). These occurrences may be identified manually using a stethoscope (to listen for the appearance and disappearance of the Korotkoff sounds), or digitally using electronic calculation.

Currently, the typical blood pressure cuff is sized for a range of arm circumferences yet contains a bladder of fixed width. This is true despite the fact that the SBP and the DBP read by the manometer vary as the ratio of bladder width to arm circumference varies. As a result, any patient whose arm circumference does not fall at the center of the range corresponding to a given cuff size will receive an inaccurate blood pressure measurement. In an attempt to mitigate this problem, the American Heart Association (AHA) has recommended using seven different cuff sizes (corresponding to seven different ranges of arm circumference) for patients ranging from pediatric to large adult. Because each cuff size contains a fixed-width bladder, however, even using the appropriately sized cuff can result in up to a plus or minus 5% error in blood pressure readings. Furthermore, because following the AHA recommendation requires measuring the patient's arm before choosing the appropriate cuff size, many practitioners (for example in clinics, wards, and/or physicians' offices) ignore the recommendation and use a standard cuff with a 12-centimeter-wide bladder in almost all blood pressure measurements. This can result in even larger errors, on the order of 5-10 mm Hg in some tests, for both the SBP and the DBP. Such errors can lead to costly misdiagnoses, especially for patients whose blood pressures are near the cutoff levels for prescribing treatment for hypertension. Further, with cutoff levels used to prescribe hypertension medication, errors in blood pressure measurements that result in high blood pressure measurements may result in patients receiving medication who do not need the medication. This results in increased costs and patients being unnecessarily medicated. Errors in blood pressure measurements that result in low blood pressure measurements may result in patients that should receive blood pressure medication not receiving such medication, thereby resulting in health complications due to untreated hypertension.

It has been suggested that the ratio of the width of the bladder covering the artery to the patient's arm circumference should be approximately in the range of 40% in order to properly occlude the artery. In addition, it has been suggested that the bladder should encircle at least about 80% of the arm and less than 100% of the arm (such that the bladder does not overlap onto itself) in order to provide accurate readings. Based on the above discussion of current practices, there exists a need for a blood pressure cuff that meets both of these requirements for accuracy while fitting patients with a range of arm circumferences.

Examples of known blood pressure cuffs can be found in US2004/092833 A1, US4,901,732A US2013/109981A1 and JP2009072278.

**SUMMARY OF THE INVENTION**

According to a first aspect, there is provided a blood pressure cuff according to claim 1. Further advantageous embodiments are according to the dependent claims.

**BRIEF DESCRIPTION OF DRAWINGS**

FIG. 1 is a schematic illustration of a blood pressure cuff with a linearly tapered bladder and an alignment tab.

FIG. 2 is a schematic illustration of the method of aligning the blood pressure cuff on a patient's arm such that the bladder contacts the patient's brachial artery at the appropriate position for an accurate reading.

FIG. 3 is a schematic illustration of a blood pressure cuff with a curved tapered bladder and an alignment tab.

FIGS. 4-6 are graphs showing the width of a bladder in relation to a lengthwise distance from an edge of the bladder using a linear taper for each of a small, medium, and large cuff.

FIGS. 7-9 are graphs showing the width of a bladder in relation to a lengthwise distance from an edge of the bladder using a curved taper for each of a small, medium, and large cuff.

## DETAILED DESCRIPTION

Specific embodiments of the present invention are now described with reference to the figures, wherein like reference numbers indicate identical or functionally similar elements. Specific embodiments are now described with reference to the figures, wherein like reference numbers indicate identical or functionally similar elements.

The following detailed description is merely exemplary in nature and is not intended to limit the invention or the application and uses of the invention. Furthermore, there is no intention to be bound by any expressed or implied theory presented in the preceding technical field, background, brief summary or the following detailed description. The terms "sphygmomanometer" and "blood pressure cuff" are used throughout this specification interchangeably, and are understood by those of ordinary skill in the art as being interchangeable terms.

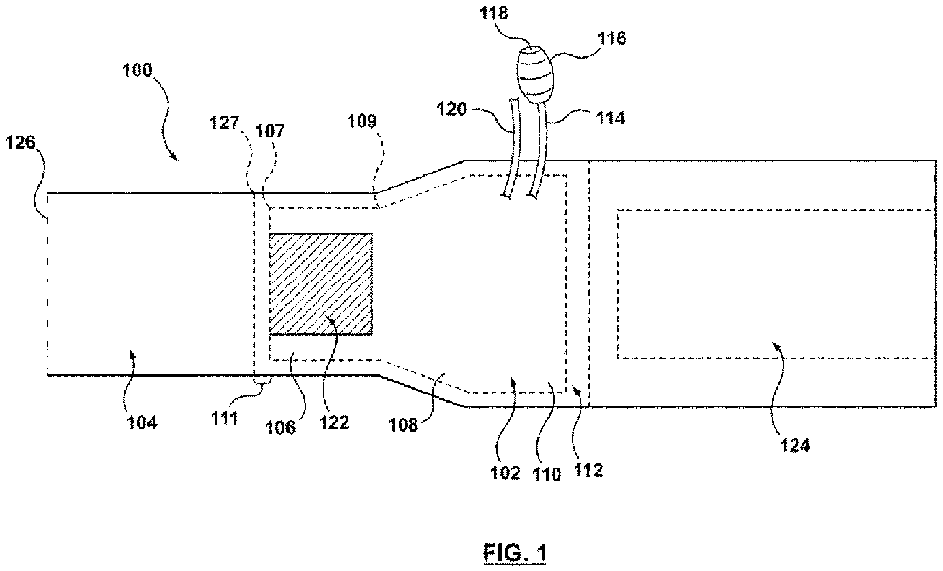

FIG. 1 illustrates a schematic view of a blood pressure cuff 100 with a tapered bladder 102 and an alignment tab 104. Bladder 102 is inflatable and includes a narrow rectangular section 106, a linearly tapered section 108, and a wide rectangular section 110. Bladder 102 may be constructed of PVC rubber, plastic, or any other similar material that is generally non-permeable to air. Bladder 102 consists of two layers, which form a pocket for holding air. Bladder 102 is shown in FIG. 1 in dashed lines because it is housed within a sleeve 112. A tube 114 is connected at a first end to the internal pocket of bladder 102 and at a second end to a squeeze ball 116. An opening 118 in ball 116 allows atmospheric air to enter ball 116. When ball 116 is squeezed, air is transferred through tube 114 and into bladder 102 to inflate bladder 102. A second tube 120 has a first end connected to the internal pocket of bladder 102 and a second end (not shown) connected to a manometer (not shown).

In the embodiment of FIG. 1, hook and loop sections 122 and 124 on opposing sides of cuff 100 are used to secure cuff 100 around the patient's arm. However, other means of fixing the cuff to itself such that it remains in a fixed position once wrapped around the patient's arm may also be used. Additionally, while FIG. 1 shows the shape of sleeve 112 generally mimicking the shape of bladder 102, sleeve 112 may be of any shape larger than bladder 102 and may use seams or other means to ensure that bladder 102 does not move significantly within sleeve 112. Sleeve 112 may be constructed of nylon or similar fabric, or of any other sufficiently flexible material. Alignment tab 104 may be constructed of the same material as sleeve 112 or of a different sufficiently flexible material. While tab 104 is shown as generally rectangular in FIG. 1, it may be of any shape and it may be wider or narrower than sleeve 112 to which it is joined.

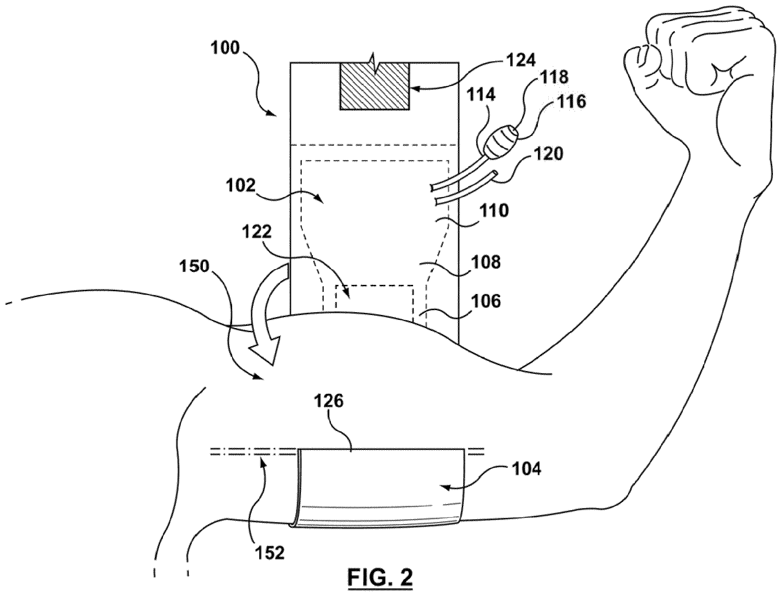

A method useful for understanding the invention is shown in fig. 2. FIG. 2 schematically illustrates the method of aligning cuff 100 with the patient's arm 150. As shown in FIG. 2, an edge 126 of tab 104 is placed tangentially to the patient's brachial artery 152. Cuff 100 is wrapped around arm 150 such that bladder 102 intersects brachial artery 152, and then cuff 100 is fixed into position by attaching hook and loop sections 122 and 124 to each other. It is important that the method of aligning cuff 100 is easy so that users properly align cuff so that the proper width of bladder 102 is aligned with the brachial artery.

Alignment tab 104 is sized according to the range of arm circumferences for which cuff 100 is intended. The length of tab 104 that wraps around patient's arm 150 is such that narrow rectangular section 106 of bladder 102 intersects brachial artery 152 if the patient's arm circumference is equal to the minimum value supported by the particular size of cuff 100. Similarly, tab 104 ensures that wide rectangular section 110 intersects brachial artery 152 if the patient's arm circumference is equal to the maximum value supported by the cuff size, and that appropriate portion of linearly tapered section 108 intersects brachial artery 152 if the patient's arm circumference falls between the limits supported by the size of cuff 100. This design ensures that the ratio of bladder width (at the point where it intersects brachial artery 152) to arm circumference is maintained at the desired width of bladder to arm circumference ratios for any arm circumference falling within the range supported by the size of cuff 100. Additionally, the lengths of tab 104 and bladder 102 are such that, for an arm circumference falling within the bounds specified by the size of cuff 100, bladder 102 encircles the patient's arm 150 without overlapping onto itself.

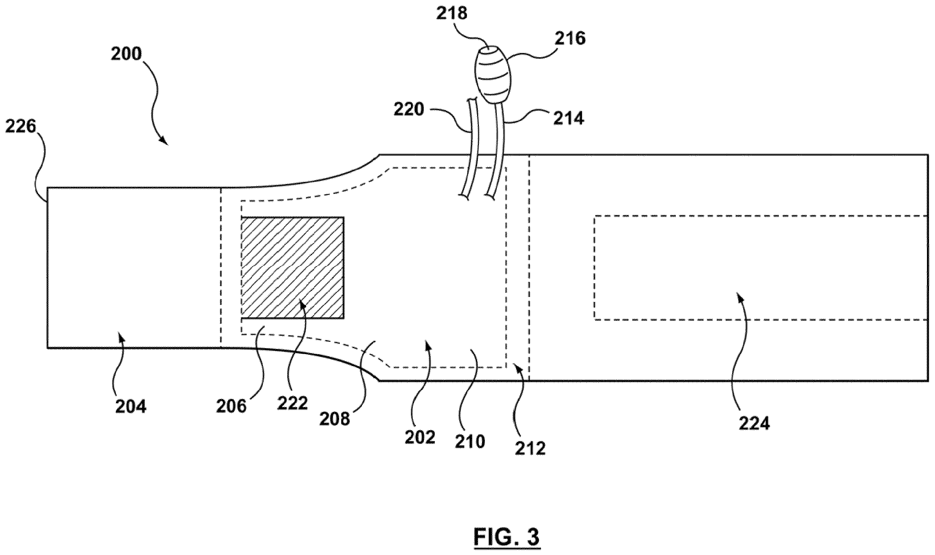

Accordingly, as can be seen in the charts provided below, the length of tab 104 and the length of the narrow rectangular section 106 of bladder 102 add up to almost the minimum arm circumference for the cuff 100. The total is not equal to minimum arm circumference because there is a distance or gap 111 from a second end 127 of tab 104 to an edge 107 of the narrow rectangular section 106 of bladder 102 of approximately 1 cm. Accordingly, the length from edge 126 of tab 104 to a transition 109 from narrow rectangular section 106 of bladder 102 to tapered section 108 of bladder 108 is approximately equal to the minimum arm circumference for the cuff. Those skilled in the art would recognize that variations in the length of tab 104, narrow rectangular section 106, and gap 111 may be made provided that the total length from edge 126 of tab 104 to transition 109 is approximately the minimum circumference for the cuff. The term "approximately" or "Substantially" as used herein means "about" or "around" and is used to accommodate manufacturing tolerances and other minor variations. Accordingly, as used herein, the term "approximately" or "substantially" means within 5%. The table below provides exemplary sizes of parts of the cuff 100 for particular ranges of arm circumferences. The reference numerals used in the chart refer the FIG. 1, but the chart applies equally to the embodiment of FIG. 3, described below. All numbers provided within the table are in units of centimeters.

| Cuff Size | Arm Circ. Range | Tab 104 Length | Narr. Rect. 106 Length | Narr. Rect. 106 Width | Taper 108 Length | Wide Rect. 110 Length | Wide Rect. 110 Width |
|---|---|---|---|---|---|---|---|
| Small | 22-26 | 19 | 2 | 10.33 | 4 | 2 | 12.29 |
| Medium | 27-34 | 24 | 2 | 12.78 | 7 | 2 | 16.23 |
| Large | 35-44 | 32 | 2 | 16.73 | 9 | 2 | 21.18 |

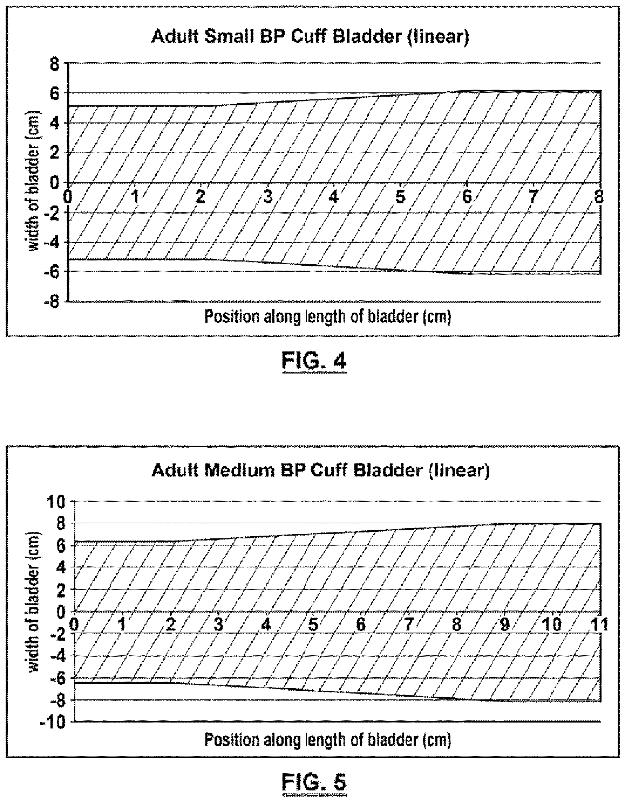

In the chart above, the taper is a linear taper. Thus, the width of the bladder along the taper length can be calculated by the commonly known formula for a line: $Y = m*X + b$, wherein m is equal to the slope and b is equal to the Y-axis intercept. In order to calculate the formula for the line of the tapered section, the bladder parameters may be laid out on a graph. Two points on the line can be determined by ½ the width of the bladder in the narrow section as the Y-axis at 2 cm along the X-axis and ½ the width of the wide section of the bladder as the Y-axis at 6 cm along the X-axis. One half of the width is used such that half of the bladder is above the X-axis and half of the bladder is below the X-axis. Solving the equation results in the line of the tapered section of the bladder of the small cuff identified above being defined as $Y = 0.245*X + 4.675$. FIGS. 4-6 show graphs of the bladder sections for small, medium, and adult cuffs with the dimensions as noted above. Further the width at the narrow rectangular section 106 and the width of the wide rectangular section 110 are each approximately calculated according to the formula: $W = (C-LOG(C))/2$; wherein C = the circumference of the patient's arm and W = the width of the tapered section for a particular circumference.

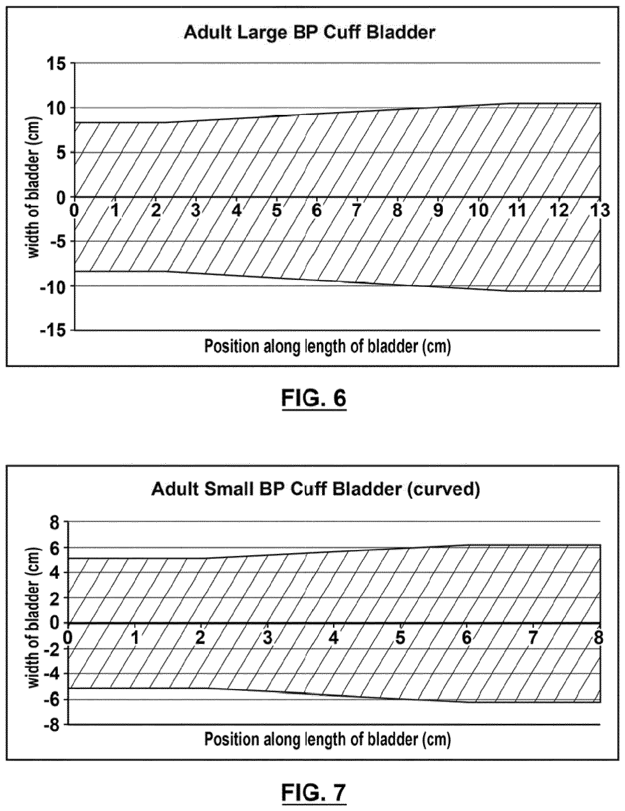

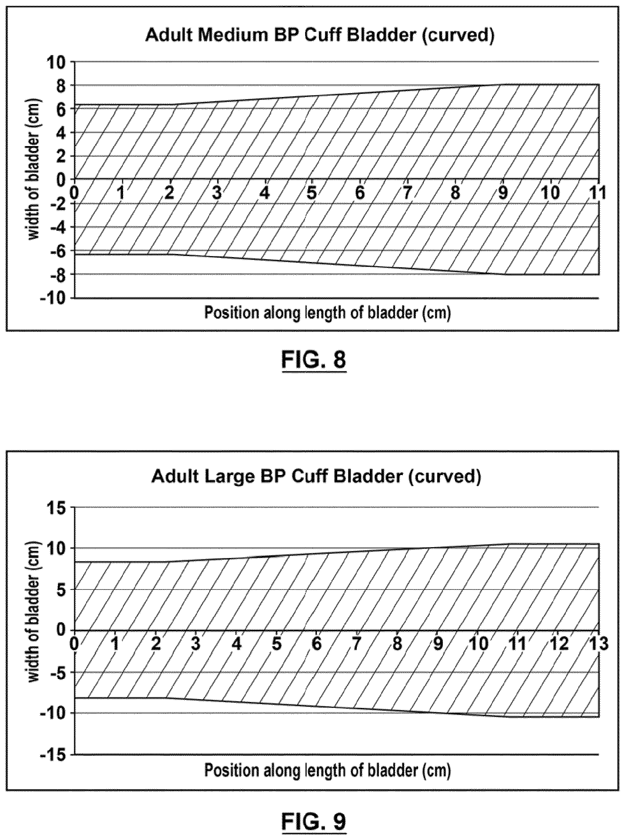

FIG. 3 illustrates a schematic view of another embodiment of a blood pressure cuff 200, which also contains an inflatable bladder 202 and an alignment tab 204. Cuff 200 of this embodiment is similar to cuff 100 except that bladder 202 includes a curved tapered section 208 between a narrow rectangular section 206 and a wide rectangular section 210. It has been found that a suitable curve is defined by the following formula: $W = (C - LOG(C))/2$; wherein C = the circumference of the patient's arm and W = the width of the tapered section for a particular circumference. Accordingly, whereas the chart above with the linear tapered section used the above formula for the width of the bladder at the narrow rectangular section and the wide rectangular section, the chart below uses the formula for each width. FIGS. 7-9 are graphs showing the bladder in accordance with the formula above. Otherwise, cuff 200 is similar to cuff 100. Bladder 202 is housed within a sleeve 212 and is inflatable via tube 214 and squeeze ball 216. A second tube 220 has a first end connected to the internal pocket of bladder 202 and a second end (not shown) connected to a manometer (not shown). The shapes and materials of bladder 202, tab 204, and sleeve 212 are as outlined above for the first embodiment. Additionally, FIG. 3 shows the inclusion of opposing hook and loop sections 222 and 224 on cuff 200, but any other means of securing cuff 200 to itself around a patient's arm may be used. Cuff 200 is aligned with the patient's arm in the same manner as is illustrated in FIG. 2 and discussed above.

Although specific embodiments of the linear taper and curved taper have been presented above, those skilled in the art would recognize that other linear tapers or curved tapers may be utilized. For example, in the linear taper embodiment described, the ratio of the width of the bladder covering the artery to the patient's arm circumference is approximately 47%. The design of the taper of the bladder may be modified to accommodate any desired ratio. For example, and not by way of limitation, a bladder of a blood pressure cuff may be designed to accommodate a 40% ratio using this following cuff and bladder dimensions (all in cm).

| Cuff Size | Arm Circ. | Tab Length | Distance from Narrow Bladder Edge | Bladder width |
|---|---|---|---|---|
| Small | 22 | 19 | 0 | 8.8 |
| Small | 22 | 19 | 1 | 8.8 |
| Small | 22 | 19 | 2 | 8.8 |
| Small | 23 | 19 | 3 | 9.2 |
| Small | 24 | 19 | 4 | 9.6 |
| Small | 25 | 19 | 5 | 10 |

(continued)

| Cuff Size | Arm Circ. | Tab Length | Distance from Narrow Bladder Edge | Bladder width |
|---|---|---|---|---|
| Small | 26 | 19 | 6 | 10.4 |
| Small | 26 | 19 | 7 | 10.4 |
| Small | 26 | 19 | 8 | 10.4 |
| Medium | 27 | 24 | 0 | 10.8 |
| Medium | 27 | 24 | 1 | 10.8 |
| Medium | 27 | 24 | 2 | 10.8 |
| Medium | 28 | 24 | 3 | 11.2 |
| Medium | 29 | 24 | 4 | 11.6 |
| Medium | 30 | 24 | 5 | 12.0 |
| Medium | 31 | 24 | 6 | 12.4 |
| Medium | 32 | 24 | 7 | 12.8 |
| Medium | 33 | 24 | 8 | 13.2 |
| Medium | 34 | 24 | 9 | 13.6 |
| Medium | 34 | 24 | 10 | 13.6 |
| Medium | 34 | 24 | 11 | 13.6 |
| Large | 35 | 32 | 0 | 14.0 |
| Large | 35 | 32 | I | 14.0 |
| Large | 35 | 32 | 2 | 14.0 |
| Large | 36 | 32 | 3 | 14.4 |
| Large | 37 | 32 | 4 | 14.8 |
| Large | 38 | 32 | 5 | 15.2 |
| Large | 39 | 32 | 6 | 15.6 |
| Large | 40 | 32 | 7 | 16.0 |
| Large | 41 | 32 | 8 | 16.4 |
| Large | 42 | 32 | 9 | 16.8 |
| Large | 43 | 32 | 10 | 17.2 |
| Large | 44 | 32 | 11 | 17.6 |
| Large | 44 | 32 | 12 | 17.6 |
| Large | 44 | 32 | 13 | 17.6 |

Those skilled in the art would recognize that variations in the desired ratio of bladder width to arm circumference, length of narrow rectangular section of the bladder, and length of the tab may be made such that the desired width of bladder corresponds to each arm circumference with the alignment method described above.

While various embodiments according to the present invention have been described above, it should be understood that they have been presented by way of illustration and example only, and not limitation. It will be apparent to persons skilled in the relevant art that various changes in form and detail can be made therein without departing from the scope of the invention as defined by the appended claims.

**Claims**

1. A blood pressure cuff (100) comprising:

an alignment tab (104) having an edge (126);
a sleeve (112); and
an inflatable bladder (102) disposed within the sleeve (112), the inflatable bladder (102) including a first rectangular section (106) having a first width, W1, a first end and a second end, and a tapered section (108) of increasing width extending from the second end of the first rectangular section (106) in a direction away from the alignment tab (104);
wherein a length from an edge (126) of the alignment tab (104) to the second end of the first rectangular section (106) is equal to a minimum arm circumference for the blood pressure cuff;
wherein the blood pressure cuff is sized for a range of arm circumferences, the range including a first circumference, C1, and a second circumference C2, wherein the second circumference is larger than the first circumference, wherein the first circumference comprises the minimum arm circumference for the blood pressure cuff and the second circumference comprises a maximum arm circumference for the blood pressure cuff;
wherein the first width equals one half of the difference of the first circumference and the log of the first circumference:

$$W_1 = (C_1 - LOG(C_1))/2;$$

wherein the inflatable bladder further includes a second rectangular section (110) having a second width, W2, larger than the first width, wherein the tapered section (108) extends between the first rectangular section (106) and the second rectangular section (110), wherein the second width equals approximately one half the difference of the second circumference and the log of the second circumference:

$$W_2 = (C_2 - LOG(C_2))/2;$$

and
wherein the first circumference and the second circumference are measured in centimeters.

2. The blood pressure cuff of claim 1, wherein the tapered section comprises a linear taper.

3. The blood pressure cuff of claim 1, wherein the tapered section comprises a curved taper.

4. The blood pressure cuff of claim 1, wherein the blood pressure cuff is configured for arm circumferences between approximately 22 cm and 26 cm and the length from the edge of the alignment tab to the second end of the first rectangular section is approximately 22 cm.

5. The blood pressure cuff of claim 1, wherein the blood pressure cuff is configured for arm circumferences between approximately 27 cm and 34 cm and the length from the edge of the alignment tab to the second end of the first rectangular section is approximately 27 cm.

6. The blood pressure cuff of claim 1, wherein the blood pressure cuff is configured for arm circumferences between approximately 35 cm and 44 cm and the length from the edge of the alignment tab to the second end of the first rectangular section is approximately 35 cm.

7. The blood pressure cuff of claim 1, wherein the tapered section comprises a taper according to the equation W = (C - LOG(C))/2, wherein C is equal to a chosen circumference within the range of arm circumferences, and wherein W is equal to the width of the tapered section at the chosen circumference.

**Patentansprüche**

1. Blutdruckmanschette (100), umfassend:

eine Ausrichtungslasche (104) mit einem Rand (126);
eine Hülse (112); und

eine aufblasbare Blase (102), die innerhalb der Hülse (112) angeordnet ist, wobei die aufblasbare Blase (102) eine erste rechteckige Sektion (106) mit einer ersten Breite, W1, ein erstes Ende und ein zweites Ende, und eine verjüngte Sektion (108) mit zunehmender Breite aufweist, die sich von dem zweiten Ende der ersten rechteckigen Sektion (106) in einer Richtung von der Ausrichtungslasche (104) weg erstreckt;

wobei eine Länge von einem Rand (126) der Ausrichtungslasche (104) zu dem zweiten Ende der ersten rechteckigen Sektion (106) gleich ist einem minimalen Armumfang für die Blutdruckmanschette;

wobei die Blutdruckmanschette für einen Bereich von Armumfängen bemessen ist, wobei der Bereich einen ersten Umfang, C1, und einen zweiten Umfang, C2, umfasst, wobei der zweite Umfang größer ist als der erste Umfang, wobei der erste Umfang den minimalen Umfang für die Blutdruckmanschette umfasst, und der zweite Umfang einen maximalen Armumfang für die Blutdruckmanschette umfasst;

wobei die erste Breite gleich ist einer Hälfte der Differenz des ersten Umfangs und des log des ersten Umfangs:

$$W_1 = (C_1 - \mathrm{LOG}(C_1))/2;$$

wobei die aufblasbare Blase ferner eine zweite rechteckige Sektion (110) mit einer zweiten Breite, W2, aufweist, die größer ist als die erste Breite, wobei sich die verjüngte Sektion (108) zwischen der ersten rechteckigen Sektion (106) und der zweiten rechteckigen Sektion (110) erstreckt, wobei die zweite Breite gleich ist ungefähr einer Hälfte der Differenz des zweiten Umfangs und des log des zweiten Umfangs:

$$W_2 = (C_2 - \mathrm{LOG}(C_2))/2;$$

und

wobei der erste Umfang und der zweite Umfang in Zentimetern gemessen werden.

2. Blutdruckmanschette nach Anspruch 1, wobei die verjüngte Sektion eine lineare Verjüngung umfasst.

3. Blutdruckmanschette nach Anspruch 1, wobei die verjüngte Sektion eine gekrümmte Verjüngung umfasst.

4. Blutdruckmanschette nach Anspruch 1, wobei die Blutdruckmanschette für Armumfänge zwischen ungefähr 22 cm und 26 cm ausgelegt ist, und die Länge von dem Rand der Ausrichtungslasche zu dem zweiten Ende der ersten rechteckigen Sektion ungefähr 22 cm beträgt.

5. Blutdruckmanschette nach Anspruch 1, wobei die Blutdruckmanschette für Armumfänge zwischen ungefähr 27 cm und 34 cm ausgelegt ist, und die Länge von dem Rand der Ausrichtungslasche zu dem zweiten Ende der ersten rechteckigen Sektion ungefähr 27 cm beträgt.

6. Blutdruckmanschette nach Anspruch 1, wobei die Blutdruckmanschette für Armumfänge zwischen ungefähr 35 cm und 44 cm ausgelegt ist, und die Länge von dem Rand der Ausrichtungslasche zu dem zweiten Ende der ersten rechteckigen Sektion ungefähr 35 cm beträgt.

7. Blutdruckmanschette nach Anspruch 1, wobei die verjüngte Sektion eine Verjüngung gemäß der Gleichung W = (C - LOG(C))/2 umfasst, wobei C gleich ist einem gewählten Umfang innerhalb des Bereichs von Armumfängen, und wobei W gleich ist der Breite der verjüngten Sektion bei dem gewählten Umfang.

**Revendications**

1. Brassard de tensiomètre (100), comprenant :

un onglet d'alignement (104) ayant un bord (126) ;
un manchon (112) ; et
une vessie gonflable (102) disposée à l'intérieur du manchon (112), la vessie gonflable (102) incluant une première section rectangulaire (106) ayant une première largeur, W1, une première extrémité et une seconde extrémité, et une section conique (108) de largeur croissante s'étendant de la seconde extrémité de la première section rectangulaire (106) dans une direction à distance de l'onglet d'alignement (104) ;
dans lequel une longueur depuis un bord (126) de l'onglet d'alignement (104) à la seconde extrémité de la première section rectangulaire (106) est égal à une circonférence de bras minimale pour le brassard de tensiomètre ;

dans lequel le brassard de tensiomètre est dimensionné pour une plage de circonférences de bras, la plage incluant une première circonférence, C1, et une seconde circonférence, C2, dans lequel la seconde circonférence est plus grande que la première circonférence, dans lequel la première circonférence comprend la circonférence de bras minimale pour le brassard de tensiomètre et la seconde circonférence comprend une circonférence de bras maximale pour le brassard de tensiomètre ;

dans lequel la première largeur est égale à une moitié de la différence de la première circonférence et du log de la première circonférence :

$$W_1 = (C_1 - LOG(C_1))/2 \; ;$$

dans lequel la vessie gonflable inclut en outre une seconde section rectangulaire (110) ayant une seconde largeur, W2, plus grande que la première largeur, dans lequel la section conique (108) s'étend entre la première section rectangulaire (106) et la seconde section rectangulaire (110), dans lequel la seconde largeur équivaut approximativement à une moitié de la différence de la seconde circonférence et du log de la seconde circonférence :

$$W_2 = (C_2 - LOG(C_2))/2 \; ;$$

et

dans lequel la première circonférence et la seconde circonférence sont mesurées en centimètres.

2. Brassard de tensiomètre selon la revendication 1, dans lequel la section conique comprend un cône linéaire.

3. Brassard de tensiomètre selon la revendication 1, dans lequel la section conique comprend un cône incurvé.

4. Brassard de tensiomètre selon la revendication 1, dans lequel le brassard de tensiomètre est configuré pour des circonférences de bras entre environ 22 cm et 26 cm, et la longueur du bord de l'onglet d'alignement à la seconde extrémité de la première section rectangulaire est d'environ 22 cm.

5. Brassard de tensiomètre selon la revendication 1, dans lequel le brassard de tensiomètre est configuré pour des circonférences de bras entre environ 27 cm et 34 cm, et la longueur du bord de l'onglet d'alignement à la seconde extrémité de la première section rectangulaire est d'environ 27 cm.

6. Brassard de tensiomètre selon la revendication 1, dans lequel le brassard de tensiomètre est configuré pour des circonférences de bras entre environ 35 cm et 44 cm, et la longueur du bord de l'onglet d'alignement à la seconde extrémité de la première section rectangulaire est d'environ 35 cm.

7. Brassard de tensiomètre selon la revendication 1, dans lequel la section conique comprend un cône selon l'équation W = (C-LOG(C))/2, dans lequel C est égal à une circonférence choisie dans la plage de circonférences de bras, et dans lequel W est égal à la largeur de la section conique à la circonférence choisie.

FIG. 1

**FIG. 2**

EP 2 997 889 B1

**FIG. 3**

EP 2 997 889 B1

**Adult Small BP Cuff Bladder (linear)**

FIG. 4

**Adult Medium BP Cuff Bladder (linear)**

FIG. 5

**FIG. 6**

**FIG. 7**

**Adult Medium BP Cuff Bladder (curved)**

FIG. 8

**Adult Large BP Cuff Bladder (curved)**

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004092833 A1
- US 4901732 A
- US 2013109981 A1
- JP 2009072278 B